(19) **Europäisches Patentamt · European Patent Office · Office européen des brevets**

(11) **EP 1 460 415 B1**

(12) **EUROPEAN PATENT SPECIFICATION**

(45) Date of publication and mention
of the grant of the patent:
**12.01.2011 Bulletin 2011/02**

(51) Int Cl.:
**G01N 27/28** (2006.01)    **G01N 27/30** (2006.01)
**G01N 27/416** (2006.01)    **B01L 3/00** (2006.01)
**C12M 1/34** (2006.01)

(21) Application number: **02803538.4**

(22) Date of filing: **19.11.2002**

(86) International application number:
**PCT/JP2002/012085**

(87) International publication number:
**WO 2003/044512 (30.05.2003 Gazette 2003/22)**

(54) **MEASUREMENT DEVICE FOR MEASURING ELECTRIC SIGNAL EMITTED BY BIOLOGICAL SAMPLE**

MESSVORRICHTUNG ZUR MESSUNG EINES VON EINER BIOLOGISCHEN PROBE EMITTIERTEN ELEKTRISCHEN SIGNALS

APPAREIL DE MESURE DESTINE A MESURER DES SIGNAUX ELECTRIQUES EMIS PAR UN ECHANTILLON BIOLOGIQUE

(84) Designated Contracting States:
**AT BE BG CH CY CZ DE DK EE ES FI FR GB GR IE IT LI LU MC NL PT SE SK TR**

(30) Priority: **19.11.2001  JP 2001353829**

(43) Date of publication of application:
**22.09.2004  Bulletin 2004/39**

(73) Proprietor: **Panasonic Corporation Kadoma-shi Osaka 571-8501 (JP)**

(72) Inventors:
• **OZAKI, Nobuhiko**
**Ikoma-shi, Nara 630-0239 (JP)**
• **OKA, Hiroaki**
**Hirakata-shi, Osaka 573-1194 (JP)**
• **SUGIHARA, Hirokazu**
**Katano-shi, Osaka 576-0054 (JP)**

(74) Representative: **Grünecker, Kinkeldey, Stockmair & Schwanhäusser Anwaltssozietät Leopoldstrasse 4 80802 München (DE)**

(56) References cited:
**DE-A1- 19 948 473     JP-A- 9 211 010
JP-A- 9 289 886     JP-A- 11 299 496
US-A- 5 674 742     US-A1- 2001 005 774**

• **MUELLER T ET AL: "3-D MICROELECTRODE SYSTEM FOR HANDLING AND CAGING SINGLE CELLS AND PARTICLES" BIOSENSORS AND BIOELECTRONICS, ELSEVIER BV, NL, vol. 14, 15 March 1999 (1999-03-15), pages 247-256, XP000912020 ISSN: 0956-5663**

# Description

## TECHNICAL FIELD

The present invention relates to a measurement device for electrophysiological evaluation of a biological sample.

## BACKGROUND ART

Conventionally, in chemical screening using a biological sample (represented by a cell), the action of a cell is monitored by fluorochrome method or a micropipette electrode method to evaluate the action and effect of a drug for the cell.

Fluorochrome methods are methods for optically measuring, using fluorochromes which specifically react with various ion concentrations and a membrane-potential-sensitive fluorochrome, the action of a cell which is influenced by a drug, i.e., the degree of change in fluorescence of the cell according to changes in the membrane potential and ion concentration of the cell. In fluorochrome methods, the step of staining a cell with a dye is necessary. Therefore, influence of a dye to a cell can not be eliminated. Moreover, in fluorochrome methods, fluorescence from a fluorochrome is weakened with time, so that detection accuracy becomes poor. This is a weak point of fluorochrome methods.

On the other hand, micropipette electrode-methods are of a method for directly measuring an electrical action of a cell, such as an intracellular potential, a current flowing through an ion channel existing in a cell membrane, a current flowing through a channel activated by a drug receptor. Therefore, with the micropipette electrode method, more detail and wider range of information about the action of a cell which is influenced by a drug can be obtained. The micropipette electrode method is a very useful method for obtaining information about the action of a cell.

In general, in an intracellular potential recording method that is one of micropipette electrode methods, a micropipette formed by stretching thermally-melt quartz glass or borosilicate glass so as to have a submicron tip diameter is inserted into a cell. Moreover, in a patch clamp method that is one of micropipette electrode methods, to locally measure a very small region of about 0.1-20 micron in the cell, a micropipette is pressed against a cell membrane so that the micropipette and the membrane are attached to each other at a predetermined angle.

### Problems to be solved

However, in a known micropipette electrode method, a technician operates a micropipette, relying on a micrograph, to insert a micropipette into a cell fixed onto a substrate, or attach a micropipette to the cell. Therefore, a highly accurate micropipette position controller is needed, and furthermore, a skill to insert a micropipette into a cell or attach a micropipette to a cell is needed.

Accordingly, the micropipette electrode methods are not suitable as a technique for screening a large amount of compounds as candidates for drugs at high speed.

In Japanese Laid-Open Patent Publication 9-289886, a cell membrane potential detector for use in a micropipette electrode method is disclosed. The cell membrane potential detector includes a protruding electrode for cell insertion, which is provided on a bottom face of a dish having a plurality of with bottom holes and serves as a micropipette, and a reference electrode provided in a side surface of each of the with bottom holes.

The cell membrane potential detector disclosed in the publication does not include means for accurately leading a cell to the protruding electrode. Therefore, a skill of a technician is needed to lead a cell to a protruding electrode.

In view of the above-described problems, the present invention has been devised to provide a measurement device which allows electrophysiological measurement of a biological sample to be performed in a simple, accurate, high-speed and automatic manner.

## DISCLOSURE OF INVENTION

A measurement device according to the present invention is a measurement device for measuring an electric signal from a biological sample and includes: a flow pass extending in a first direction; a pair of introduction orifices connected to both ends of the flow pass, respectively; a concave well formed in the flow pass; a reference electrode formed in the flow pass; at least a microelectrode formed in the well; a first communicating tube connected to the well and extending in a second direction different from the first direction; a second communicating tube connected to other part of the well than part of the well to which the first communicating tube is connected and extending in a third direction different from the first and second directions; first and second resistance baths connected to the first and second communicating tubes, respectively; and first and second resistors arranged in the first and second communicating tubes, respectively, or the first and second resistance baths, respectively.

In the measurement device of the present invention, when a current is applied to the first and second resistors, the first and second resistors consume electric energy and generate heat. The measurement device is used with the flow pass filled with an electrolyte solution and a medium capable of changing an interfacial tension with the electrolyte solution in each of the first and second resistance baths added thereto, and currents having different current values are applied to the resistors, respectively. Thus, the interfacial tension between the medium shut into each of the first and second resistance baths and the electrolyte solution in each of the first and second communicating tubes is decreased in proportion to the quantity of heat generated by the resistors. As a result, the electrolyte solution in the first and second communicating tubes moves from the resistance bath of a higher temperature to the resistance bath of a lower temperature. This causes a local flow of the electrolyte solution in the well. By using the local flow, a biological sample is accurately and quickly led to the microelectrode, and then electrophysiological measurement of the biological sample can be performed. Therefore, with the measurement device of the present invention, a highly accurate position controller, which has been required in a known device of this type, and a skilled operation are no longer necessary.

It is preferable that the first and second communicating tubes are arranged so as to have line symmetry with respect to the flow pass.

Thus, calculation of the direction in which and the speed at which the biological sample is led by the local flow of the electrolyte solution in each of the first and second communicating tubes can be simplified very much.

It is preferable that said at least a microelectrode includes a protruding end portion. Thus, the microelectrode can be inserted into the biological sample.

It is preferable that the first and second communicating tubes are arranged so that a straight line extending along the first communicating tube and a straight line extending along the second communicating tube intersect with each other toward said at least a microelectrode.

Thus, the local flow of the electrolyte solution in the well can be generated toward the microelectrode.

Said at least a microelectrode may be provided plural in number.

Thus, a plurality of biological samples can be fixed to the plurality of microelectrodes, respectively, so that all of the biological samples can be measured at the same time or each of the biological samples can be separately measured.

It is preferable that a surface area of the reference electrode is larger than a total surface area of the plurality of microelectrodes.

Thus, measurement stability against disturbances such as a solution flow can be improved.

The measurement device of the present invention may further include: a first substrate; and a second substrate formed on the first substrate, and have a structure in which the first substrate includes the well, the reference electrode, the microelectrode, the first and second communicating tubes, the first and second resistance baths, and the first and second resistors, and the second substrate includes a concave portion which serves as the flow pass when the second substrate is provided on the first substrate and extends in the first direction and cavities which serve as the pair of introduction orifices when the second substrate is provided on the first substrate and are formed in both ends of the concave portion, respectively.

It is preferable that the second substrate is formed of a transparent material.

Thus, the flow pass can be observed from the outside.

It is preferable that the second substrate is formed of a semiconductor device. Thus, the first and second resistance baths and the first and second communicating tubes can be formed using a semiconductor fabrication technique known by a person skilled in the art. Specifically, if wet etching is used, by taking advantage of plane-directional selectivity, the first and second resistance baths and the first and second communicating tubes can be formed with high accuracy.

It is preferable that a volume of the first communicating tube is smaller than one fifth of a volume of the first resistance bath and a volume of the second communicating tube is smaller than one fifth of a volume of the second resistance bath.

Thus, by appropriately adjusting the interfacial tension in each of the first and second resistance baths, the location of an interface of the electrolyte solution can be controlled in a simple manner so that the interface is located in a desired position in each of the first and second communicating tubes.

It is preferable that a surface of the flow pass has been subjected to hydrophilic processing.

Thus, a liquid can be made to flow in a very small flow pass.

It is preferable that each of the first and second resistors has a comb shape.

Thus, even in a narrow region, each of the first and second resistors can have a great length, so that a large resistance value can be obtained.

It is preferable that the measurement device of the present invention further includes a third communicating tube connected to other part of the well than the parts of the well to which the first and second communicating tubes are connected and extending in a fourth direction different from the second and third directions; a third resistance bath connected to the third communicating tube; and a third resistor arranged in the third communicating tube or the third resistance bath.

Thus, the position of the biological sample contained in the electrolyte solution can be freely controlled. Furthermore, the degree of freedom of position control of the biological sample contained in the electrolyte solution is increased, so that the microelectrode can be freely formed in the well. That is, the degree of freedom of design of the inside of the well is improved.

It is preferable that the first and second communicating tubes are arranged so that a line extending along each said tubes extends toward said at least a microelectrode and has an opening toward said at least a microelectrode, and the third communicating tube is arranged so that the end portion of said at least a microelectrode is located substantially on a straight line extending along the third communicating tube.

Thus, in the well, the local flow of the electrolyte solution can be generated so as to flow toward any direction of front, back, right and left with respect to the microelectrode.

The measurement device may further include: another flow pass extending in the first direction; another pair of introduction orifices connected to both ends of said another flow pass, respectively; another concave well formed in said another flow pass; another reference electrode formed in said another flow pass; at least another microelectrode formed in said another well; another first communicating tube connected to said another well and extending in the second direction different from the first direction; another second communicating tube connected to other part of said another well than part of said another well to which said another first communicating tube is connected; another first resistance bath and another second resistance bath connected to said another communicating tube and said another communicating tube, respectively; and another first resistor and another second resistor arranged in said another first communicating tube and said another second communicating tube, respectively, or in said another first resistance bath and said another second resistance bath, respectively.

Thus, multiple measurements can be performed at the same time.

A measurement method according to the present invention is a method for measuring an electrical signal from a biological sample, the method comprising the steps of: a) preparing a measurement device including a flow pass extending in a first direction, a pair of introduction orifices connected to both ends of the flow pass, respectively, a concave well formed in the flow pass, a reference electrode formed in the flow pass, at least a microelectrode formed in the well, a first communicating tube connected to the well and extending in a second direction different from the first direction, a second communicating tube connected to other part of the well than part of the well to which the first communicating tube is connected and extending in a third direction different from the first and second directions, first and second resistance baths connected to the first and second communicating tubes, respectively, and first and second resistors arranged in the first and second communicating tubes, respectively, or the first and second resistance baths, respectively; b) introducing an electrolyte solution into the flow pass by reduction of a pressure in the flow pass to fill the flow pass; c) returning the pressure in the flow pass to an atmosphere pressure; d) arranging the biological sample in the well; e) closing up the flow pass; f) applying a current to the first or second resistor to bring said at least a microelectrode in contact with the biological sample; and g) measuring change in an electrical signal from the biological sample.

According to the measurement method of the present invention, when electrophysiological measurement of a biological sample led by an electrode, a highly accurate position controller and skilled operation, which have been conventionally needed, are no longer necessary. That is, electrophysiological measurement of a biological sample can be performed in a simple, accurate, high-speed and automatic manner.

The measurement method of the present invention may further include after the step f), the step h) of applying an electrical pulse to said at least a microelectrode.

Thus, an electrical reaction of a biological sample due to electric stimulation can be measured with high sensitivity.

The measurement method may further include the step i) of administering a drug from one of the pair of introduction orifices.

Thus, an electrical reaction of a biological sample to a drug can be measured in a simple manner.

## BRIEF DESCRIPTION OF DRAWINGS

FIG. **1** is a diagram illustrating the structure of a measurement device according to EMBODIMENT 1 of the invention.

FIG. **2** is a cross-sectional view taken along the line **III-III** of FIG. **1**.

FIG. **3** is a perspective view of the measurement cell seen from the arrow A of FIG. **1**.

FIGS. **4(a)**, **4(b)** and **4(c)** are schematic diagrams illustrating the operation of a measurement device of EMBODIMENT 1.

FIG. **5** is a perspective view of the measurement cell seen from the arrow A of FIG. **1**.

FIGS. **6(a)** and **6(b)** are schematic diagrams illustrating the outline of the principle on which the introduction of a cell into an electrode is based.

FIG. **7** is a diagram illustrating the structure of a measurement device according to EMBODIMENT 2 of the invention.

FIG. **8** is a cross-sectional view taken along the line **III-III** of FIG. **1**.

FIG. **9** is a perspective view of the measurement cell seen from the arrow A of FIG. **1**.

FIG. **10** is a flow chart illustrating a measurement method according to EMBODIMENT 4 of the present invention.

FIG. **11** is a graph showing the action potential of a nerve cell of lymnaea stagnalis when current stimulation was applied to the cell, measured using the measurement device of EMBODIMENT 1.

FIG. **12** is a graph showing the action potential of an artery smooth muscle cell when carbachol was added to the cell, measured using the measurement device of EMBODIMENT 1.

FIG. **13** is a histogram of standard deviation per 10 ms for the action potential of an artery smooth muscle cell when carbachol was added to the cell, measured using the measurement device of EMBODIMENT 1.

**BEST MODE FOR CARRYING OUT THE INVENTION**

Hereinafter, embodiments of the present invention will be described with reference to the accompanying drawings.

(EMBODIMENT 1)

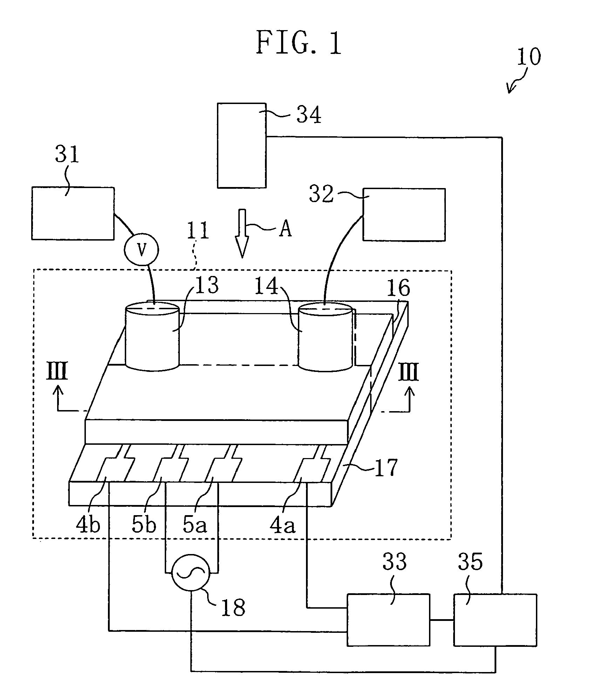

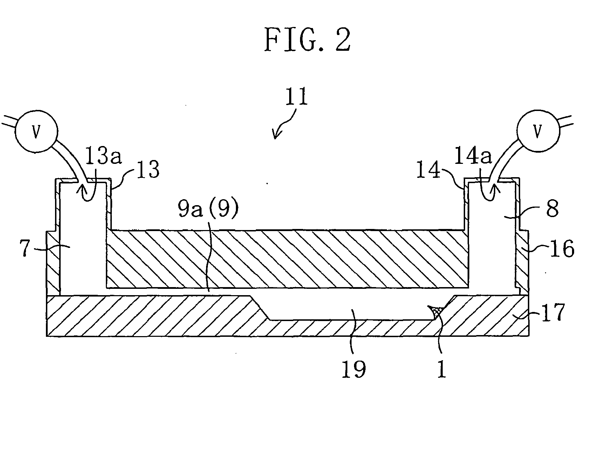

FIG. **1** is a diagram illustrating the structure of a measurement device according to this embodiment. FIG. **2** is a cross-sectional view taken along the line **III-III** of FIG. **1**.

As shown in FIG. **1**, a measurement device **10** according to this embodiment includes a measurement cell **11**, a current application section **18**, pumps **31** and **32**, a measuring section **33**, an imaging section **34**, and a calculator **35**.

The measurement cell **11** includes a substrate **17**, a substrate **16** provided on the substrate **17**. The substrates **16** and **17** are bonded to each other. As shown in FIG. **1**, terminals **4a**, **4b**, **5a** and **5b** are provided on the substrate **17**.

The current application section **18** is, as shown in FIG. **1**, electrically connected to the terminals **5a** and **5b** provided on the substrate **17**.

The pumps **31** and **32** are, as shown in FIG. **1**, connected to opening portions **13a** and **14a** formed in an inlet connector **13** and an outlet connector **14**, respectively.

The measuring section **33** is connected to the terminals **4a** and **4b**, and the calculator **35** is electrically connected to the measuring section **33**.

The imaging section **34** is arranged so as to be located directly on the measurement cell **11**. As shown in FIG. **1**, the imaging section **34** and the current application section **18** are electrically connected to the calculator **35** as necessary.

Next, the detailed structure of the measurement cell **11** will be described.

As shown in FIG. **1**, the inlet connector **13** and the outlet connector **14** are provided on the substrate **16** constituting a measurement cell. In this embodiment, as shown in FIGS. **1** and **2**, the inlet connector **13** and the outlet connector **14** are attached to the substrate 16 with an adhesive agent or the like. However, the present invention is not limited thereto, but the inlet connector **13** and the outlet connector **14** may be provided so as to make one unit together with the substrate **16**. Moreover, as shown in FIG. **2**, the insides of the inlet connector **13** and outlet connector **14** are cavities **7** and **8**, which pass through the substrate **16**. Furthermore, the opening portions **13a** and **14a** are provided in respective upper portions of the inlet connector **13** and the outlet connector **14**, respectively. The cavities **7** and **8** of the insides of the inlet connector **13** and the outlet connector **14** are connected to the pumps **31** and **32**, respectively, through tubes or the like. The cavities **7** and **8** of the insides of the inlet connector **13** and the outlet connector **14** are connected to the pumps **31** and **32**, respectively, via a valve V.

Moreover, as shown in FIG. **2,** a concave portion **9a** is formed in the substrate **16**. Thus, when the measurement cell **11** is formed by sticking the substrates **16** and **17** to each other, a flow pass **9** is formed in the measurement cell **11**.

The substrate **17**, as shown in FIG. **2**, includes a concave well **19** and a protruding microelectrode **1** provided on a side wall surface of the well **19**.

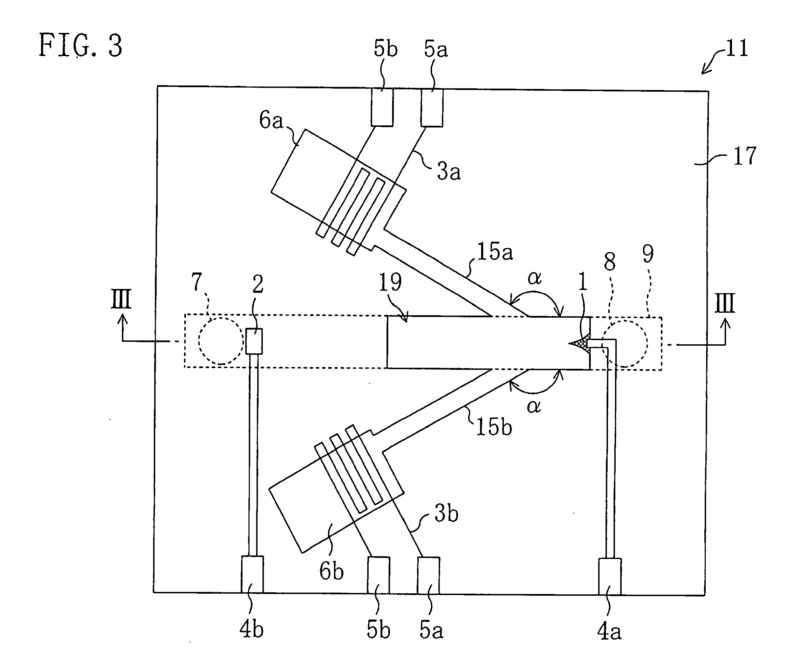

More details of the structure of the measurement cell **11** will be described with reference to the FIG. **3**, in addition to the FIGS. **1** and **2**. FIG. **3** is a perspective view of the measurement cell **11** seen from the arrow **A** of FIG. **1**. Note that a cross-sectional view taken along the line **III-III** of FIG. **3** corresponds to the cross-sectional view taken along the line **III-III** of FIG. **1**, i.e., the cross-sectional view of FIG. **2**. Moreover, in FIG. **3**, the substrate **16** of the measurement cell **11** is omitted.

As shown in FIGS. **2** and **3**, the substrate **16** is attached to the substrate **17** so that the cavities **7** and **8** are located over both ends of the flow pass **9**, respectively.

As shown in FIG. **3**, on the substrate **17** of the measurement cell **11**, provided are the concave well **19**, the protruding microelectrode **1** connected to the terminal **4a** and provided on a side wall surface of the well **19**, communicating tubes **15a** and **15b** connected to both side surfaces of the well **19** (i.e., the flow pass **9**), respectively, so as to face to each other, resistance baths **6a** and **6b** connected to the communicating tubes **15a** and **15b**, respectively, thin resistors **3a** and **3b** arranged in the resistance baths **6a** and **6b**, respectively, and a reference electrode **2** connected to the terminal **4b**.

The communicating tubes **15a** and **15b** are arranged so as to have line-symmetry with respect to the flow pass **9** (i.e., the well **19**). Moreover, assume that an angle between the flow pass **9** (i.e., the well **19**) and the communicating tube **15a** or **15b** is an angle α. The communicating tubes **15a** and **15b** are arranged so that the angle α is an obtuse angle at any time. That is, the communicating tubes **15a** and **15b** are arranged so that a straight line extending along the communicating tube **15a** and a straight line extending along the communicating tube **15b** intersect with each other toward the microelectrode **1**.

Moreover, in this embodiment, in the same manner as the communicating tubes **15a** and **15b**, the resistance baths **6a** and **6b** are arranged so as to have line-symmetry with respect to the flow pass **9** (i.e., the well **19**) and also the thin resistors **3a** and **3b** are arranged so as to have line-symmetry with respect to the flow pass **9**.

Each of the communicating tubes **15a** and **15b** has a very small cross-sectional area, compared to the resistance baths **6a** and **6b**. The resistance baths **6a** and **6b** have an arbitrary shape.

The reference electrode **2** is arranged in the vicinity of the inlet **7** of the flow pass **9** formed in the substrate **16**. Note that the reference electrode **2** may be located in any location in which the reference electrode **2** can be in contact with a liquid, and is preferably arranged in the flow pass **9** and the well **19**. Accordingly, the reference electrode **2** can be formed on the substrate **17**. However, when simpleness of fabrication is taken into consideration, the reference electrode **2** is preferably provided on the substrate **16**.

As shown in FIGS. **1** and **3**, the microelectrode **1** and the reference electrode **2** are connected to the measuring section **33** via the terminals **4a** and **4b**, respectively. As the measuring section **33**, a current/voltage amplifier including a bridge-balancing circuit, a current stimulation circuit, and a capacity compensation mechanism and the like is preferably used in the micropipette electrode method.

As shown in FIGS. **1** and **3**, the thin resistors **3a** and **3b** are connected to the current application section **18** via the terminals **5a** and **5b**, respectively. The current application section **18** applies a desired current to the thin resistors **3a** and **3b**.

As the substrate **17**, a semiconductor substrate formed of a silicon substrate or the like is preferably used. If a semiconductor substrate is used as the substrate **17**, the resistance baths **6a** and **6b** and the communicating tubes **15a** and **15b** can be formed by a semiconductor fabrication technique known by a person skilled in the art. Specifically, the resistance baths **6a** and **6b** and the communicating tubes **15a** and **15b** are preferably formed by wet etching. This is because when wet etching is used, by taking advantage of plane-directional selectivity, the substrate **17** (i.e., a measurement cell) having a highly accurate and fine structure can be obtained.

Note that either dry etching or wet etching is used, the above-described resistance baths and communicating tubes can be formed. When wet etching is performed, as an etchant, an isotropic etchant or an anisotropic etchant is selectively used according to need.

As the substrate **16**, a transparent substrate is preferably used so that the flow pass **9** can be observed from the imaging section **34**. The respective cavities **7** and **8** of the inlet and outlet connectors **13** and **14** provided on the substrate **16** and the flow pass **9** can be formed using a processing technique (specifically, cutting process, grinding process or the like) known by a person skilled in the art or a semiconductor fabrication technique known by a person skilled in the art. Specifically, as the substrate **16**, a glass substrate is preferably used. Thus, the respective cavities **7** and **8** of the inlet and outlet connectors **13** and **14** provided on the substrate **16** and the concave portion **9a** can be formed using a semiconductor fabrication technique known by a person skilled in the art with high accuracy.

The microelectrode **1** is formed of a conductive material. Specifically, in this embodiment, the microelectrode **1** is formed in a manner in which a single crystalline silicon is made into a shape having a micro-size protruding portion by under etching. A surface of the micro-size silicon protruding portion is coated with a metal thin film and then an edge portion of the protruding portion is removed. Thereafter, the micro-size silicon protruding portion is coated with an insulator. In this manner, the microelectrode **11** has a sharp protruding portion having a tip diameter of about 0.1 μm.

In this embodiment, the reference electrode **2**, the terminals **4a**, **4b**, **5a** and **5b**, an interconnect for connecting the microelectrode **1** and the terminal **4a**, an interconnect for connecting the reference electrode **2** and the terminal **4b**, and the resistors **3a** and **3b** are formed using a thin film forming technique such as vacuum evaporation and sputtering. However, method for forming these members are not limited thereto, but these members may be formed by other methods known by a person skilled in the art.

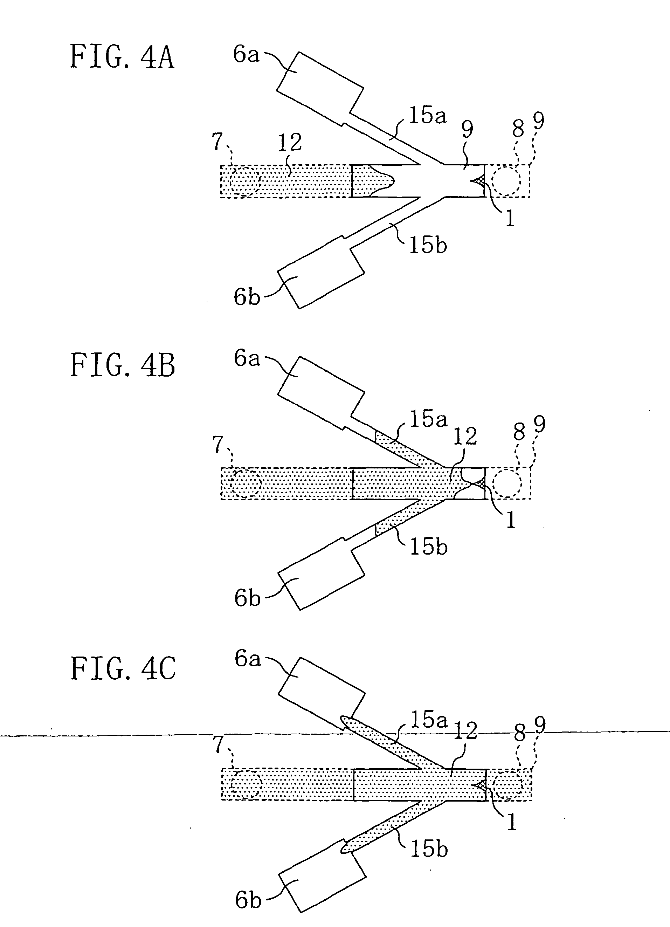

Next, a solution introduction method of the measurement device **11** of this embodiment will be described with reference to FIG. **4**.

FIGS. **4(a)**, **4(b)** and **4(c)** are schematic diagrams illustrating a solution introduction method of a measurement device **11** of this embodiment.

First, a valve **V** connected to the connector having the inlet **8** of the flow pass **9** of the device is closed and then suction is performed by a pump **32** connected to the cavity **8** (i.e., the outlet connector **14**) serving as an outlet of the flow pass **9**. In this manner, a pressure in each of the flow pass **9**, the communicating tubes **15a** and **15b**, and the resistance baths **6a** and **6b** is reduced to a pressure **Pd** slightly lower than an atmosphere pressure **P1**. Subsequently, as shown in FIG. **4(a)**, the valve **V** is opened to the air, so that an electrolyte solution **12** is injected into the flow pass **9** from the pump **31** connected to the valve **V** through the cavity **7** (i.e., the inlet connector **13**).

At this time, a pressure in each of the resistance baths **6a** and **6b** connected to the flow pass **9** through the communicating tubes **15a** and **15b**, respectively, is the pressure **Pd** lower than the atmosphere pressure **P1**. Accordingly, as shown in FIG. **4(b)**, the electrolyte solution **12** injected into the flow pass **9** fills the well **19**, as well as the communicating tubes **15a** and **15b**, to flow toward the cavity **8** (i.e., the outlet connector **14**) serving as an outlet.

Subsequently, the electrolyte solution **12** reaches the cavity **8** as the outlet of the flow pass **9**, the suction of the pump **32** connected to the outlet connector **14** is stopped. Then, the cavity **8** serving as the outlet is opened to the air by valve operation or the like to return the pressure in the flow pass **9** back to the atmosphere pressure **P1**. At this time, the pressures in the resistance baths **6a** and **6b** are still substantially at **Pd**. Thus, the electrolyte solution **12** filling the communicating tubes **6a** and **6b** flows towards the resistance baths **6a** and **6b**.

When each of the pressures in the resistance baths **6a** and **6b** becomes equal to **P1**, the flow of the electrolyte solution **12** stops, as shown in FIG. **4(c)**. At this time, the injection of the electrolyte solution **12** is stopped and the valve **V** connected to the cavity **7** (i.e., the inlet connector **13**) serving as an inlet is closed.

Next, a biological sample **21** (a cell in this embodiment) is introduced in the electrolyte solution **12**. In this embodiment, when the biological sample **21** is introduced, properties obtained according to the shape of the slow pass **9** are used.

The flow pass **9** is a space whose height is very small, as shown in FIG. **2**. There is a characteristic in which with a small space filled with a liquid like the flow pass **9**, when another liquid further flows therein, the liquid introduced later tends to be a laminar flow. When a liquid flowing in the flow pass **9** flows as a laminar flow, the liquid flowing substantially does not mix with the liquid with which the well **19** is filled and passes in an upper stream. In this embodiment, a sample solution containing the biological sample **21** is injected through the cavity **7** (i.e., the inlet connector **13**) from the pump **31** connected to the valve **V** into the flow pass **9** filled with the electrolyte solution **12**. In this case, the biological sample **21** is monitored, and when the biological sample **21** reaches the upper portion of the well **19**, the injection of the sample solution is stopped. Thus, the laminar flow disappears in the flow pass **9** and diffusion of the electrolyte solution **12** becomes superior, so that the biological sample **21** is introduced into the well **19**.

Note that in this embodiment, the introduction of the biological sample **21** has been described. However, this embodiment is not limited thereto, but introduction and removal of an agent can be performed in the exactly same manner as described above.

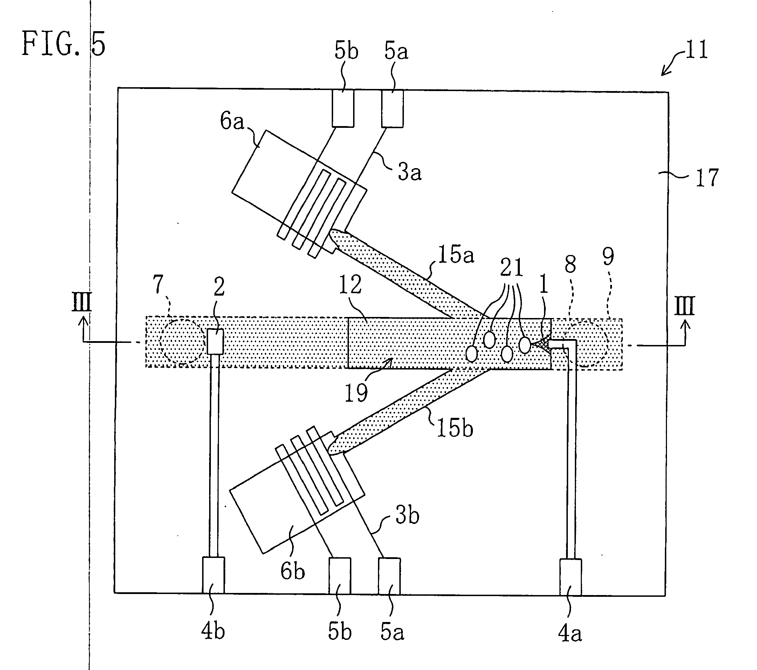

Finally, as shown in FIG. **5**, the flow pass **9**, the well **19** and the communicating tubes **15a** and **15b** are filled with the electrolyte solution **12**.

Note that in this embodiment, as the electrolyte solution **12**, an extracellular fluid for measuring an intracellar potential of a cell, i.e., the biological sample **21**, is used. As the extracellular fluid, a saline solution containing as a main component NaCl of 20 mM to 400 mM, various kinds of nutrients, growth factors, culture medium containing an antibiotic or the like can be used.

Specifically, the measurement device **10** is designed so that the volumes of the resistance baths **6a** and **6b** are much larger than those of the communicating tubes **15a** and **15b**, respectively, or the volume of the well **19**. Therefore, by appropriately adjusting the pressure in the resistance baths **6a** and **6b**, a vapor-liquid interface can be controlled to be located at a desired position in the communicating tubes **15a** and **15b** in a simple manner. More specifically, the volumes of the resistance baths **6a** and **6b** are preferably five times as large as or more than those of the communicating tubes **15a** and **15b**.

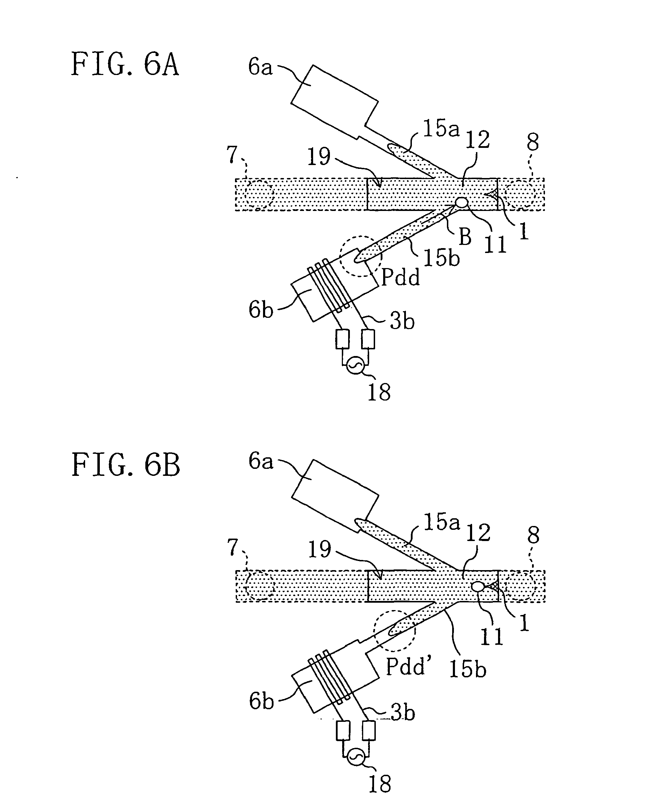

Next, the outline of the principle on which the introduction of a cell into an electrode performed in the measurement device **10** of this embodiment is based will be described with reference to FIG. **6**. FIGS. **6(a)** and **6(b)** are schematic diagrams illustrating the outline of the principle on which the introduction of a cell into an electrode is based. Here, a case in which a current is applied to the thin film resistor **3b** arranged in the resistance bath **6b** of the measurement device **10** will be described as an example.

First, when a current is applied to the thin film resistor **3b** by the current application section **18**, an electrical energy **W** represented by Equation 1 is consumed in the thin resistor **3b**.

$$[\text{Equation 1}] \qquad W = RI^2$$

where **R** is a resistance value [$\Omega \cdot m$] of the thin film resistor **3b** and **I** is a current [A] flowing in the thin film resistor **3b**.

The consumed electrical energy **W** is transformed into heat energy. This heat energy expands air in the resistance base **6b**. Accordingly, as shown in FIG. **6(a)**, a pressure in the resistance bath **6b** is made to be **Pdd**, and at the same time, the interfacial tension of the vapor-liquid interface of the communicating tube **15b** connected to the resistance bath **6b** is reduced. Since the interfacial tension of the vapor-liquid interface in the cavity **8** serving as the outlet of the flow pass **9** is not changed, an equilibrium in the interfacial tension of the vapor-liquid interface between the communicating tube **15b** and the communicating tube **15a** is lost. Accordingly, the electrolyte solution **12** flows from the resistance bath **6b** to the well **19** in the direction which the arrow **B** of FIG. **6(a)** points (i.e., toward the resistance bath having a lower temperature). The biological sample **21** moves according to the flow of the electrolyte solution **12** is led by the microelectrode **1** to be inserted.

Next, when current supply to the resistor **6b** is stopped, part of the heated air in the resistance bath **6b** is returned to an ambient temperature and the pressure in the resistance bath **6b** becomes **Pdd'**. An equilibrium between the interfacial tensions is achieved again, so that the biological sample **21** is held as shown in FIG. **6(b)** at a location to which the biological sample **21** has moved. The volume of the communicating tube **15b** is very small and the electrolyte solution **12** has a small heat capacitance. Therefore, the electrolyte solution **12** moves very quickly.

In this embodiment, when the activity of a cell is measured, the cavity **7** and the cavity **8** which serve as an inlet and an outlet, respectively, are closed. Thus, a distance **x** ($\mu$m) by which the electrolyte solution **12** has been moved through the communication tube **15b** is in proportion to a difference between the interfacial tension of the vapor-liquid interface in the communicating tube **15a** and the interfacial tension of the vapor-liquid interface in the communicating tube **15b**. Therefore, by controlling a current value **Ib** and a current variation speed Δ**Ib** (A/s) of a current applied to the thin film resistor **3b** or by controlling a difference between current values **Ia** and **Ib** of currents applied to the thin film resistors **3a** and **3b**, respectively, and a difference between current variation speeds Δ**Ia** and Δ**Ib**, a difference between the interfacial tensions is adjusted. Thus, the distance **x** (μm) of a movement of the vapor-liquid interface in the communicating tube **15a** and a moving speed Δ**x** (μm/s) for the vapor-liquid interface in the communicating tube **15a** are adjusted.

In this embodiment, the case in which the vapor-liquid interface in the communicating tube **15b** is moved has been described. However, when the vapor-liquid interface in the communicating tube **15a** is moved, the exactly same operation is performed.

In this embedment, while the biological sample **21** is being monitored, the location of the biological sample **21** is feedback-controlled. An image of the biological sample **21** is digitized by the imaging section **34** (e.g., CCD) through the transparent substrate **16**, the digitized image is input into the calculator **35**, and then the amount of a current to be made to flow in each of the thin film resistors **3a** and **3b** is calculated based on image information such as the amount of a movement of the biological sample **21**. The measurement device **10** may further include means for automatically applying a current of a current value obtained by the calculation to the current application section **18**. Needless to say, the above-described operation may be performed in a manual manner by a technician. However, by automating the above-described operation, measurement can be preformed in a more simple manner.

As has been described, in the measurement device **10** of this embodiment, the current values **Ia** and **Ib** applied to the thin film resistors **3a** and **3b**, respectively, and the current variation speeds Δ**Ia** and Δ**Ib** (A/s) are controlled to adjust the difference between the interfacial tensions of the vapor-liquid interfaces in the resistance baths **6a** and **6b** (or the communicating tubes **15a** and **15b**), so that the distance and speed of a movement of the vapor-liquid interface in each of the communicating tubes **15a** and **15b** is adjusted.

Thus, in the measurement device **10** of this embodiment, a local flow of the electrolyte solution **12** can be generated in the well **19**, the biological sample **21** is accurately and quickly led to the microelectrode **1** by using the local flow, and then an electrophysiological measurement of the biological sample **21** can be performed. Therefore, a highly accurate position controller and skilled operation, which have been needed in the known device of this type, are no longer necessary.

Moreover, with the measurement device **10** of this embodiment, a chemical substance such as a nerve stimulator and a chemical synapse messenger can be administered to a cell **21** in the well **19**, so that a highly accurate measurement as an intracellular recording method in which ion channel properties and chemical synapse of a cell are measured can be performed in a simple manner.

Specifically, in the measurement device **10**, the communicating tubes **15a** and **15b** are arranged so as to substantially have line-symmetry with respect to the flow pass **9** (i.e., the well **19**). Thus, a local flow of the electrolyte solution **12** generated in the well **19** can be generated between the communicating tubes **15a** and **15b** in bilateral directions (i.e., in the right and left directions when viewed from the microelectrode **1**).

Furthermore, as shown in FIG. 3, assume that an angle between the flow pass **9** (i.e., the well **19**) and each of the communicating tubes **15a** and **15b** is an angle α. The communicating tubes **15a** and **15b** are arranged so that the angle α is an obtuse angle at any time. Thus, a local flow of the electrolyte solution **12** in the well **19** can be generated toward the microelectrode **1** at any time.

As can be understood from the description above, in the measurement device **10** of this embodiment, a local flow of the electrolyte solution **12** can be two-dimensionally generated in a horizontal plane.

Moreover, in this embodiment, the communicating tubes **15a** and **15b** are arranged so as to substantially have line-symmetry with respect to the flow pass **9** (i.e., the well **19**). Thus, calculation of a difference between the interfacial tensions of the vapor-liquid interfaces in the communicating tubes **15a** and **15b** can be simplified very much.

Furthermore, in this embodiment, the resistance baths **6a** and **6b** are arranged so as to have line-symmetry with respect to the flow pass **9** (i.e., the well **19**) in the same manner as the communicating tubes **15a** and **15b**. Thus, calculation of a difference between the interfacial tensions of the vapor-liquid interfaces between the resistance baths **6a** and **6b** (or the communicating tubes **15a** and **15b**) can be further simplified. Therefore, the difference between the interfacial tensions of the vapor-liquid interface can be simplified in a very simple manner.

Furthermore, in the measurement device **10** of this embodiment, the reference electrode **2** is arranged in the vicinity of the inlet **7** of the flow pass **9** formed on the substrate **16**. However, the reference electrode **2** may be located in any place as long as the reference electrode **2** is in contact with the electrolyte solution **12** when the flow pass **9** is filled with the electrolyte solution **12**. Specifically, to perform an accurate measurement, the reference electrode **2** is preferably provided in the outside of the well **19** in which a local flow of the electrolyte solution **12** is generated.

Next, application examples for using the measurement device **10** of this embodiment will be described.

(Application Example 1: Measurement of the action potential of a cell stimulated by a current)

The measurement device **10** of this embodiment can measure an electric signal from a cell by a voltage clamp method and a current clamp method in the same manner as the intracellular recording method using the known glass micropipette.

Specifically, as described above, the cell **21** is led to the microelectrode **1**, the microelectrode **1** is inserted into the cell **21**, and then the potential of the microelectrode **1** with respect to the reference electrode **2** is measured in the measuring section **33**.

Moreover, a current pulse generator is provided in the measuring section **33** and the microelectrode **1** is connected to the current pulse generator. Then, a current stimulation is given from the microelectrode **1** and the cell **21** via the terminal **4a**. Thus, the depolarization of the cell due to the current stimulation, a temporary action potential of the cell and the like can be calculated by the calculator **35**.

(Application Example 2: Measurement of the membrane potential of a cell stimulated by drug administration)

The measurement device **10** of this embodiment can be used for measuring ion channel properties of a cell. Measurement of channel properties of a cell is performed by administering a chemical substance such as a nerve stimulator and a chemical synapse messenger to the cell **21** by the inlet connector **13** (cavity **7**), the outlet connector **14** (cavity **8**) and pumps **31** and **32** connected to the inlet connector **13** and the outlet connector **14**, respectively.

In another way, a drug can be also injected by an inkjet method using a voltage device, a method using a fine pressure pump or a microfluid drive unit, a method disclosed in Japanese Laid-Open Patent Publication No. 10-337177, an electroporation method or the like.

For example, electroporation is a technique in which a DC pulse is applied between a drug reservoir and a target to which a drug is to be injected, thereby injecting a desired amount of the drug. Specifically, after the flow pass **9** has been filled with the electrolyte solution **12** and the cell **21** and a drug has been added to the electrolyte solution **12** in the well **19**, a DC pulse is applied between the reference electrode **2** and the microelectrode **1** to inject a desired amount of the drug into the cell **21**.

In this embodiment, the microelectrode **1** is provided on a wall surface of the well **19**, but may be provided on a bottom surface of the well **19**.

Moreover, in this embodiment, the microelectrode **1** may be provided plural in number per the reference electrode **2** in the well **19**. When the microelectrode **1** is provided plural in number, the plurality of microelectrodes **1** may be connected to the terminal **4a** or may be connected to a plurality of terminals, respectively. However, when the microelectrode **1** is provided plural in number, the surface area of the reference electrode **2** is preferably larger than that of the total area of the plurality of microelectrodes **1**. This is for improving measurement stability against disturbances such as a solution flow.

When the microelectrode **1** is provided plural in number in the well **19**, with a cell **21** fixed to each of the microelectrodes **1**, all of cells can be measured at a time or each cell can be measured separately. More specifically, a cell as a biological sample is not necessarily alive. Then, assume that, for example, the survival rate of cells is 90%. In the measurement device **10** of this embodiment having the structure in which the microelectrode **1** is provided plural in number in the well **19**, stochastically, data for substantially nine cells can be obtained by one measurement. This shows that this embodiment also has the effect of improving measurement efficiency.

In this embodiment, the connectors **13** and **14** are attached to the substrate **16** by an adhesive agent or the like. However, the present invention is not limited thereto, but the connectors **13** and **14** may be formed as one unit together with the substrate **16**.

Materials used as the substrate **16** and the substrate **17** includes: semiconductor materials represented by a single crystalline silicon, amorphous silicon, silicon carbide, silicon oxide and silicon nitride; composite materials of these semiconductor materials represented by SOI (silicon on insulator); inorganic materials represented by glass, quartz glass, alumina, sapphire, ceramics, forsterite and photosensitive glass; organic materials represented by polyethylene, ethylene, polypropylene, polyisobutylene, polyethylene terephthalate, unsaturated polyester, fluorine-containing-resin, polyvinyl chloride, polyvinylidene chloride, polyvinyl acetate, polyvinyl alcohol, polyvinyl acetal, acrylic resin, polyacrylonitrile, polystyrene, acetal resin, polycarbonate, polyamide, phenolic plastic, urea resin, epoxy resin, melamine resin, styrene acrylonitrile copolymer, acrylonitrile butadiene-styrene copolymer, polyphenylene oxide, and polysulfone; a novolac resin-diazonaphthoquinone (DNQ) type photosensitive material containing novolac resin as base resin; polymetyl metacrylate (PMMA); a copolymer containing PMMA; polyethylene sulfone; polyhexafluorobutyl methacrylate; polymethyl isopropenyl ketone (PMIPK); polydimethyl siloxane (PDMS); and photosensitive materials containing an azide compound containing cyclopolyisoprene as a base polymer (e.g., 2-6-di-4-methyl cychlo hexanon). However, materials used as the substrate **16** and the substrate **17** are not limited thereto. Among these, amorphous silicon, single crystalline silicon, or glass is preferably used as the substrates **16** and **17**.

Note that the concave portion **9a** located in the substrate **16** and constituting the flow pass **9** and the surface of the wall **19** located in the substrate **17** are preferably subjected to hydrophilic treatment. Thus, a fluid can flow into a very small flow pass **9**.

Each of the microelectrode **1** and the reference electrode **2** is formed of an electrode material, such as precious metal materials including platinum, platinum black, gold, palladium, rhodium, silver, mercury, tungsten, and compounds of these metals, or a carbon material represented by graphite, glassy carbon, pyrolytic graphite, carbon paste, and carbon fiber. However, materials for the microelectrode **1** and the reference electrode **2** are not limited thereto. Note that the microelectrode **1** and the reference electrode **2** may be coated by a conductive high polymer. Thus, a stable fixed electrode can be formed. Moreover, the electrodes can be coated by a monomolecular film.

The thin film resistors **3a** and **3b** can be formed of a material such as precious metals including platinum, platinum black, gold, palladium, rhodium, silver, mercury, tungsten, nickel, titanium and compounds of these metal, a carbon material represented by graphite, glassy carbon, pyrolytic graphite, carbon paste, and carbon fiber, and other materials such as Si, Ge, ZnO, CdS, $TiO_2$, GaAs, and titanium. However, materials for the thin film resistors **3a** and **3b** are not limited thereto. Note that as the microelectrode **1** and the reference electrode **2**, the thin film resistors **3a** and **3b** may be coated by conductive macromolecules. Thus, a stable thin film resistor can be formed. Moreover, the resistors can be coated by a monomolecular film.

In this embodiment, the resistance baths **6a** and **6b** are filled with air. However, fillers to fill the resistance baths **6a** and **6b** are not limited to air, but any gases and liquids which can change an interfacial tension with the electrolyte solution **12** may be used.

Note that in this embodiment, the thin film resistors **3a** and **3b** are arranged in the resistance baths **6a** and **6b**, respectively. However, if the thin film resistors **3a** and **3b** are arranged in the communicating tubes **15a** and **15b**, the measurement device **10** of this embodiment can be operated in much the same manner. Moreover, each of the thin film resistors **3a** and **3b** preferably has a comb shape. Thus, even in a narrow region, each of the first and second resistors can have a great length, so that a large resistance value can be obtained.

Moreover, in this embodiment, the pumps **31** and **32** are used. However, the present invention is not limited thereto, but syringes may be used instead of the pump **31** and **32**.

(EMBODIMENT 2)

In this embodiment, a measurement cell **11'** which can be used instead of the measurement cell **11** in the measurement device **10** of EMBODIMENT 1 will be described with reference to the accompanying drawings. Note that each member also shown in EMBODIMENT 1 is identified by the same reference numeral for the purpose of simplicity.

The measurement cell **11'** of this embodiment has substantially the same appearance as that of the measurement cell **11** of EMBODIMENT 1. Thus, in this embodiment, a case where the measurement cell **11** is replaced by the measurement cell **11'** will be described.

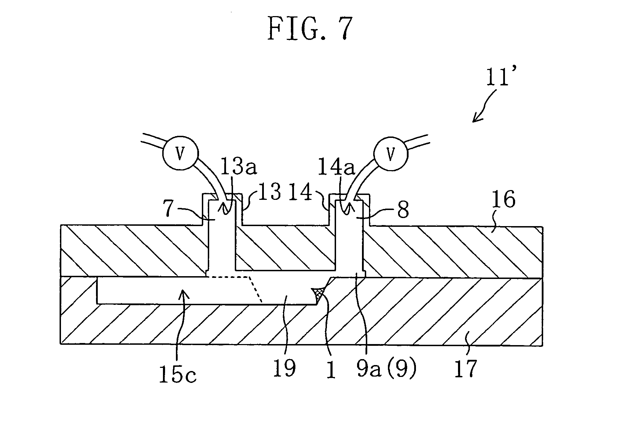

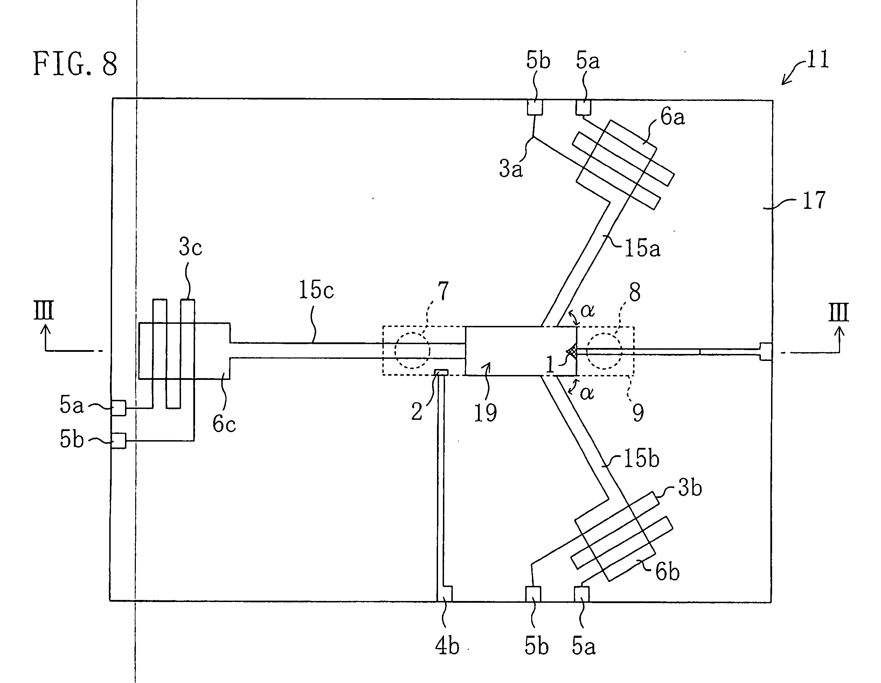

FIG. **7** is a cross-sectional view taken along the line **III-III** of FIG. **1**. FIG. **8** is a perspective view of the measurement cell **11'** seen from the arrow **A** of FIG. **1**. Note that a cross-sectional view taken along the line **III-III** of FIG. **8** corresponds to the cross-sectional view of FIG. **7**. Moreover, in FIG. **8**, the substrate **16** of the measurement cell **11'** is omitted.

The measurement cell **11'** has substantially the same structure as that of the measurement cell **11** of EMBODIMENT 1. However, as shown in FIGS. **7** and **8**, the measurement cell **11'** is different from the measurement cell **11** in that a communicating tube **15c** connected to the well **19** (i.e., the flow pass **9**), a resistance bath **6c** connected to the tube **15c**, and a thin film resistor **3c** arranged in the resistance bath **6c** are provided.

Thus, in the measurement cell **11'**, a local flow of the electrolyte solution **12** can be generated so as to flow toward any direction of front, back, right and left in a horizontal plane in the well **19** (i.e., totally freely in a two dimensional manner). Therefore, with the measurement cell **11'** of this embodiment, the position of the biological sample **21** contained in the electrolyte solution **12** can be controlled more freely than in the case where the measurement cell **11** of EMBODIMENT 1 is used.

Furthermore, the degree of freedom of position control of the biological sample **21** contained in the electrolyte solution **12** is increased, so that the microelectrode **1** can be freely formed in the well **19**. That is, the degree of freedom of design for the inside of the well **19** is improved.

In the measurement cell **11** of this embodiment, the communicating tube **15c** connected to the well **19** (i.e., the flow pass **9**) so as to face the microelectrode **1**, the resistance bath **6c** connected to the communicating tube **15c**, and the thin film resistor **3c** arranged in the resistance bath **6c** are provided. Thus, a local flow of the electrolyte solution **12** in the well **19** can be generated in the direction toward the microelectrode **1**.

Moreover, as shown in FIG. **8**, assume that an angle between the flow pass **9** (i.e., the well **19**) and each of the communicating tubes **15a** and **15b** is an angle α. The communicating tubes **15a** and **15b** are arranged so that the angle α is an acute angle at any time. That is, the communicating tubes **15a** and **15b** are arranged so that a line extending along each of the tubes **15a** and **15b** extends toward the microelectrode **1** and each of the tubes **15a** and **15b** has an opening toward the microelectrode **1**. Thus, a local flow of the electrolyte solution **12** in the well **19** can be generated to move away from the microelectrode **1** at ay time.

Furthermore, the communicating tubes **15a** and **15b** are arranged so as to have line-symmetry with respect to the flow pass **9** (i.e., the well **19**). Thus, a local flow of the electrolyte solution **12** in the well **19** can be generated in bilateral directions between the communicating tubes **15a** and **15b** (i.e., in the right and left directions viewed from the microelectrode **1**).

As can be seen from the description above, the measurement cell **11'** of this embodiment has a structure having a high symmetry as shown in FIG. **8**. Thus, calculation of differences among the interfacial tensions of the vapor-liquid interfaces in the resistance baths **6a**, **6b** and **6c** (or the communicating tubes **15a**, **15b** and **15c**) can be simplified. Therefore, the differences among the interfacial tensions can be adjusted in a simple manner.

More specifically, it is preferable that in the measurement cell **11'** of this embodiment, the angle between the flow pass **9** (i.e., the well **19**) and each of the communicating tubes **15a** and **15b** is about 60 degrees and the communicating tube **15c** is provided so as to face the microelectrode **1**. Thus, calculation of differences among the interfacial tensions of the vapor-liquid interfaces in the resistance baths **6a**, **6b** and **6c** (or the communicating tubes **15a**, **15b** and **15c**) can be simplified most. Therefore, the differences among the interfacial tensions can be adjusted in a very simple manner.

Note that in this embodiment, a structure in which the communicating tubes extending in three different directions, the resistance baths connected to the communicating tubes, respectively, and the resistors connected to the communicating tubes, respectively, are provided is used. However, an additional communicating tube extending in a different direction from any one of the three directions, an additional resistance bath connected to the communicating tube, and an additional resistor connected to the communicating tube may be further provided. Thus, the biological sample **21** may be led in the direction in which the additional communicating tube extends in a simple manner. That is, the biological sample **21** can be led in an increased number of directions in a simple manner. Therefore, the biological sample **21** can be led to the microelectrode **1** with higher accuracy.

(EMBODIMENT 3)

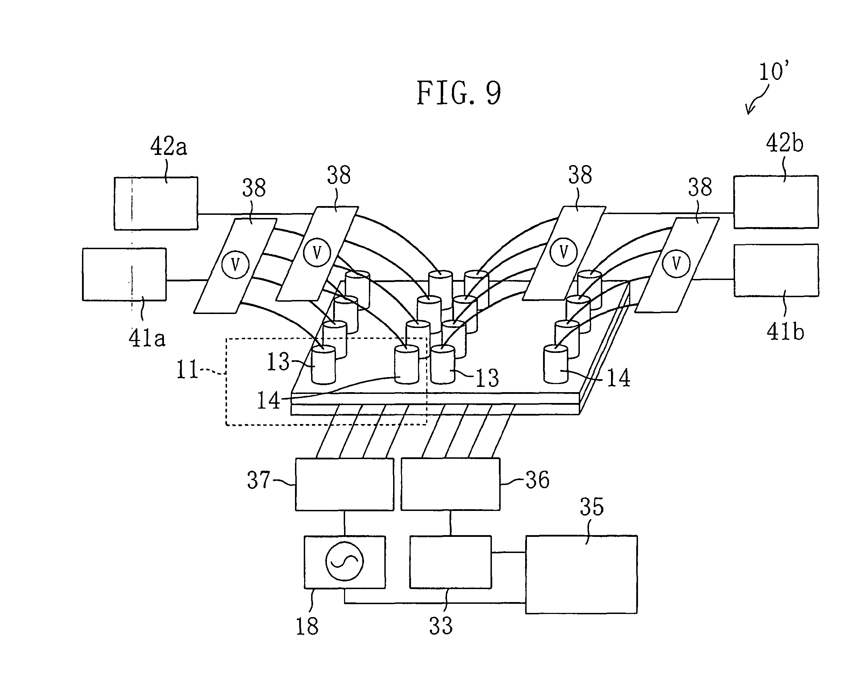

FIG. **9** is a diagram illustrating the structure of a measurement device of this embodiment.

As shown in FIG. **9**, the measurement device **10'** of this embodiment includes the measurement cell **11** or the measurement cell **11'**, as the measurement device **10** of EMBODIMENT 1 or EMBODIMENT 2. More specifically, in the measurement device **10'** of this embodiment, the measurement cell **11** or the measurement cell **11'** is provided plural in number. The plurality of measurement cells **11** or **11'** are integrated in a matrix pattern. The structure of the measurement cell **11** or **11'** is exactly the same as that of EMBODIMENT 1 or EMBODIMENT 2.

Inlet connectors **13** and outlet connectors **14** provided in each of the plurality of measurement cells **11** or **11'** are connected to flow pass switchers **38** each including a valve and a pump. A structure is used in which the inlet connectors **13** and the outlet connectors **14** are made to be interchangeably used as outlets and as inlets by the flow pass switcher **38**.

Furthermore, in the measurement device **10'** of this embodiment, fluid drive units **41a**, **41b**, **42a** and **42b** connected to the flow pass switchers **38**, respectively, are provided. As the fluid drive units **41a**, **41b**, **42a** and **42b**, for example, pumps, air compressors, syringes, or the like are used.

Moreover, in the measurement device **10'**, the current application section **18** and the measurement section **33** can be switched by multiplexers **36** and **37**. The operations of the current application section **18**, the measurement section **33** and the multiplexers **36** and **37** are controlled by the calculator **35**.

With the measurement device **10'** of this embodiment, multiple measurements can be performed at a time.

(EMBODIMENT 4)

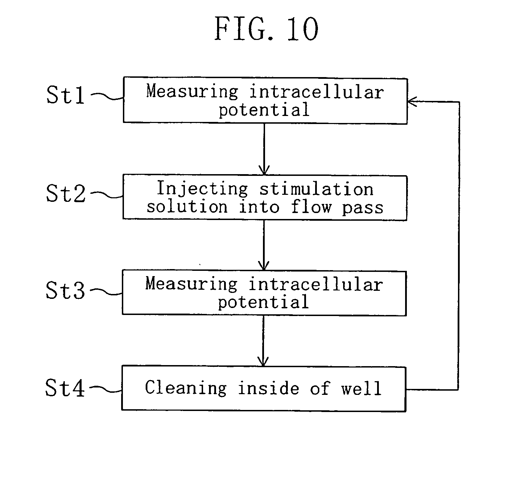

In this embodiment, a measurement method performed using the measurement device **10** of EMBODIMENT 1 will be described with reference to FIG. **10**. FIG. **10** is a flow chart describing the measurement method of this embodiment.

First, as shown in FIG. **10**, in Step **St1**, after the cell **21** has been introduced into the well **19** by the method using the laminar flow described in EMBODIMENT 1 and the position of the cell **21** has been controlled and fixed to the microelectrode **11**, an intracellular potential is measured.

Next, as shown in FIG. **10**, in Step **St2**, the valve **V** is switched so that a stimulating solution is injected from the inlet connector **13** into the flow pass **9**. When the stimulating solution reaches the well **19**, the injection of the stimulating solution is stopped to stop the flow of a liquid in the flow pass **9**. Thus, the stimulating solution is diffused in the well **19** to stimulate the cell **21** fixed to the microelectrode **11**.

Next, as shown in FIG. **10**, in Step **St3**, the intracellular potential is measured again.

Next, as shown in FIG. **10**, in Step **St4**, the inside of the well **19** is cleaned. Specifically, the valve **V** is switched so that a cleansing liquid is injected from the inlet connector **13** into the flow pass **9**. When the cleansing liquid reaches the well **19**, the injection of the cleansing liquid is stopped. Thus, the cleansing liquid is diffused in the well **19**. Subsequently, the cleansing liquid is exhausted from the outlet connector **14**. Specifically, in this step, the operations of injecting the cleansing liquid and exhausting the cleansing liquid are repeated. Thus, the well **19** is cleaned.

Next, as shown in FIG. **10**, Steps **St1** through **St4** are repeated as the concentration of the stimulator contained in the stimulating solution is gradually increased. Thus, the correlation between the concentration of the stimulator and the intracellular potential can be examined.

Moreover, by introducing an antagonist into the well **9** in the same manner as introduction of the stimulator before Step **St2**, the correlation between the antagonist and the intracellular potential can be examined.

Examples of the present invention will be described hereinafter. The following examples are described for the purposed of emplifying the present invention and do not limit the present invention.

- Examples-

Hereinafter, examples of measurement of an isolated cell as a biological sample using the measurement device **10** of EMBODIMENT 1 will be described.

(EXAMPLE 1)

*Fabrication of measurement device*

A glass plate having a thickness of 2 mm was used as a material for a substrate **16**. On a surface of the grass plate measuring 60 mm wide by 100 mm long, a concave portion **9a** was formed so as to have a width of 150 $\mu$m, a length of 3 mm and a depth of 70 $\mu$m using a $\varnothing$100 $\mu$m end mill. Thereafter, the concave portion **9a** was carefully polished. A through hole having a diameter of 1.5 mm serving as an inlet or an outlet was formed in each of end portions of the concave portion **9a**.

A single crystalline silicon wafer was used as a material for a substrate **17**. On a surface of the substrate **17**, by photolithography and anisotropic etching, formed were a well **19** having an inverted trapezoid shape, a width of 150 $\mu$m, a length of 500 $\mu$m and a depth of 30 $\mu$m, resistance baths **6a** and **6b** each having an inverted trapezoid shape, a width of 400 $\mu$m, a length of 1000 $\mu$m and a depth of 30 $\mu$m, and communicating tubes **15a** and **15b** each having an inverted triangular prism shape, a width of 10 $\mu$m, a length of 500 $\mu$m and a depth of <u>7.1 $\mu$m</u>. Specific fabrication process steps are as follows.

After having been baked at 120 °C for 20 minutes, the single crystalline wafer was sufficiently dried and then spin coating was performed at 1000 rpm for 5 seconds to form a photoresist over a surface of the single crystalline silicon wafer. Thereafter, the substrate was pre-baked at 90°C for10 minutes. The well was then mask-patterned by exposure, developed and cleaned using a developer for exclusive use in patterning, and wet-etched to complete the well **19**, the resistance baths **6a** and **6b** and the communicating tubes **15a** and **15b**. In this case, a mixed solution of 24 wt% KOH and IPA (isopropyl alcohol) as a surfactant was used as an etchant. The mixed solution was circulated using a pump and the temperature of the solution was controlled to be 73 °C $\pm$ 3 °C. Under this condition, etching was performed for 35 minutes.

A microelectrode **1** was formed by performing under etching directly under a circular etching mask using a single crystalline silicon as a material. The microelectrode having an approximately cone shape with a sharp tip, an concave outer surface, a tip neighborhood diameter of about 1 $\mu$m, a bottom surface portion with a 16 $\mu$m diameter, and a height of about 8 $\mu$m was obtained.

Thereafter, the surface of the microelectrode was subjected to sputtering to dispose a tungsten film thereon, and then the electrode was coated with polyimide, except for the tip, to provide an insulation.

Thereafter, thin film resistors **3a** and **3b** and respective lead interconnects thereof, a reference electrode **2** and a lead interconnect thereof, and a lead interconnect from the microelectrode **1** were formed on the substrate **17**. To describe simply, a platinum film of a thickness of 0.5 $\mu$m was formed over the substrate **17** by a vacuum deposition method. And then, other part of the platinum film than part thereof in which the electrode, resistors and interconnects were located was removed. Note that each of the interconnects had a width of 80 $\mu$m and each of the thin film resistors **3a** and **3b** had a comb shape having a width of 25 $\mu$m.

Then, the substrate **16** and the substrate **17** were bonded to each other by anodic bonding to form a closed flow pass **9**. Anodic bonding was performed with a direct voltage of 600 V applied at a temperature of 400-600 °C for 10 minutes. Thereafter, an inlet connector **13** and an outlet connector **14** were connected to the through holes, respectively, each being formed in each of both ends of the concave portion **9a** of the substrate **16**. Subsequently, a compressor was connected to the connector connected to the outlet of the flow pass **9** via a three-way cock and a fitting joint.

(EXAMPLE 2)

*Measurement of the action potential of a nerve cell*

Pressure in part of the flow pass **9** of the measurement device **10** located closer to the outlet connector **14** was reduced to be a slightly lower pressure than an atmosphere pressure, and then culture medium (40 mM NaCl, 1.7 mM KCl, 4.1 mM CaCl$_2$, 1.5 mM MgCl$_2$, 5 mM glucose, 5 mM HEPES, pH 7.9) for lymnaea stagnalis was injected into the flow pass **9** from the inlet connector **13** at a flow rate of 10-100 $\mu$l/min using a peristaltic pump connected to the inlet connector **13**. At this time, it was observed that the medium flew into the resistance baths **6a** and **6b** through the communicating tubes **15a** and **15b**.

Then, after having confirmed that the lymnaea stagnalis medium was injected to fill the outlet connector **14**, the operation of reducing pressure by suction from the outlet connector **14** was stopped and the three-way cock was opened to return the pressure in the part to the atmosphere pressure. At this time, the medium further flew into the communicating tubes **15a** and **15b**, so that a vapor-liquid interface was located in each of a connection portion between the communicating tube **15a** and the resistance bath **6a** and a connection between the communicating tube **15b** and the resistance bath **6b**.

A ganglion taken from lymnaea stagnalis was shaken in a 1 mg/ml protease type 14 (SIGMA, P-5147) solution at 37 °C for 25 minutes to isolate a nerve cell.

The obtained nerve cell of the lymnaea stagnalis was suspended into the lymnaea stagnalis medium and then carefully introduced into the flow pass **9** from the inlet connector **13**. The flow pass **9** was then observed under a microscope. When the valve **V** connected to the inlet connector **13** and the outlet connector **14** was closed at the moment when the lymnaea stagnalis nerve cell reached an upper portion of the well **19**, introduction of the nerve cell into the well **19** was confirmed.

Subsequently, when a current of 10 mA p-p (mili-ampere peak to peak) was applied to each of the thin film resistors **3a** and **3b** for 30 seconds, the nerve cell moved in the well **19** to be inserted into the microelectrode **1**. At this time, a rectangular wave current stimulation pulse of 10 nA p-p was applied to the microelectrode **1** for 1 ms and an intracellular potential was measured by a voltage clamp method. The intracellular potential was recorded for 60 times at intervals of 310 seconds and with a sampling frequency of 50 kHz.

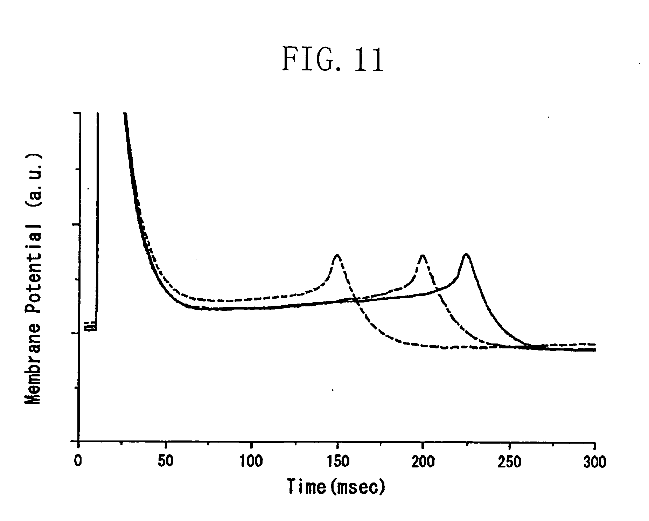

Next, measurement was performed by applying current stimulation, i.e., a rectangular current pulse of 8 nA p-p for 1 ms. Results of three measurements are shown in FIG. **11**. Note that in all of the measurements in this example, bridge balance was taken and capacitance compensation of a device was performed. Moreover, a structure was made so that measurement data passed through a 5 kHz, 4th Butterworth type low-pass filter, and then input into a calculator.

As shown in FIG. **11**, a potential response of the cell due to current stimulation was observed in a period between 150 ms and 250 ms and it was shown that the action potential of a cell can be detected by the method of this embodiment in the same manner as in a known intracellular recording method.

(EXAMPLE 3)

As the biological sample **21**, rat artery smooth muscles (SM) were used. As a solution to be injected into the measurement device **10**, DMEM culture medium to which 10% FBS (fetal bovine serum) was added was used. An SM cell was inserted into the microelectrode **1** by performing the same operation as that of EXAMPLE 2, and then an intracellular potential when 100 $\mu$M carbachol, which was an analog of acetylcholine, was introduced as a solution was measured by a voltage clamp method.

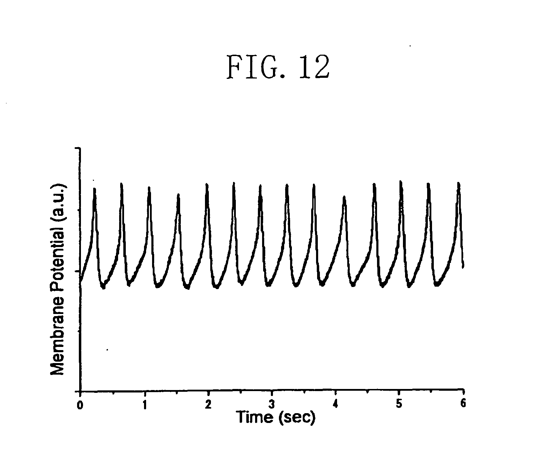

Results of measurement of the spontaneous action potential obtained when carbachol was administered are shown in FIG. **12**. As shown in FIG. **12**, the action potential in response to carbachol was detected. The results were similar to those obtained by the known intracellular recording method.

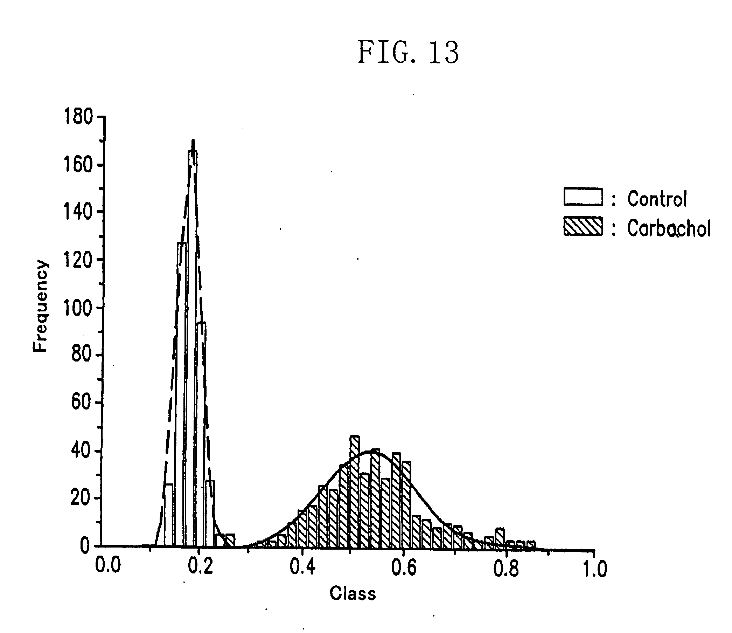

Furthermore, **FIG. 13** shows a histogram of standard deviation per 10 ms for data obtained by signal-processing data obtained by the known intracellular recording method. FIG. **13** is a histogram of standard deviation per 10 ms before and after a carbachol administration. The mean value and half width of the graph, obtained by approximating the histogram before and after a carbachol administration with a normal distribution are 0.185 and 0.04, respectively, before the administration and 0.545 and 0.21, respectively, after the administration. This shows that the mean value of the standard deviation per 10 ms was increased after the administration of carcachol. The reason for the increase was that by the administration of carbachol, ion channels of the lymnaea stagnalis nerve cell was activated, and changes in conductance due to switching of the activated ion channels is expressed by voltage change. From FIG. **13**, it was confirmed that with the measurement device **10**, ion channel properties for a nerve stimulator can be determined as in the same manner in the known intracellular recording method.

In this manner, with the measurement device **10**, it is possible to measure the action of a cell according to switching of a channel in a simple manner by an ion channel activation degree extracting method without performing the known intracellular recording method. Accordingly, by comparing, using the measurement device **10**, absolute values of channel activation degrees before and after drug administration to a cell or for an administration dosage, or increase and decrease in the channel activation degrees, measurement of cell channel activation degrees and qualitative and quantitative classification of effects of a drug can be performed.

It can be seen from the results obtained in EXMPLE 2 and EXMPLE 3 that the measurement device **10** can high-sensitively obtain by the microelectrode **1** a cell membrane potential and a temporary action potential from the cell **21** lead by the microelectrode **1**.

As has been described, according to the present invention, a measurement device which allows electrophysiological evaluation of a biological sample to be performed in a simple, accurate, high-speed and automatic manner can be provided.

**INDUSTRIAL APPLICABILITY**

Measurement device and method according to the present invention is used for performing electrophysiological evaluation of a biological sample, represented by chemical screening and the like.

**Claims**

1.   A measurement device (10) for measuring an electric signal from a biological sample, comprising:

   a flow pass (9) extending in a first direction;
   a pair of introduction orifice (13a,14a) connected to both ends of the flow pass, respectively;
   a concave wen (19) formed in the flow pass;
   a reference electrode (2) formed in the flow pass;

at least a microelectrode (1) formed in the well;
a first communicating tube (15a) connected to the well and extending in a second direction different from the first direction;
a second communicating tube connected to another part of the well than that part of the well to which the first communicating tube is connected and extending in a third direction different from the first and second directions;
first and second resistance baths (6a,6b) connected to the first and second communicating tubes, respectively; and
first and second resistors (3a, 3b) arranged in the first and second communicating tubes, respectively, or the first and second resistance baths, respectively.

2. The measurement device of claim 1, wherein the first and second communicating tubes are arranged so as to have line-symmetry with respect to the flow pass.

3. The measurement device of claim 1, wherein the first and second communicating tubes are arranged so that a straight line extending along the first communicating tube and a straight line extending along the second communicating tube intersect with each other toward said at least a microelectrode.

4. The measurement device of claim 1, wherein said at least a microelectrode includes a protruding end portion.

5. The measurement device of claim 1, wherein said at least a microelectrode is provided plural in number.

6. The measurement device of claim 5, wherein a surface area of the reference electrode is larger than a total surface area of the plurality of microelectrodes.

7. The measurement device of claim 1, further comprising:

   a first substrate; and
   a second substrate formed on the first substrate, wherein the first substrate includes the well, the reference electrode, the microelectrode, the first and second communicating tubes, the first and second resistance baths, and the first and second resistors, and
   wherein the second substrate includes a concave portion which serves as the flow pass when the second substrate is provided on the first substrate and extends in the first direction and cavities which serve as the pair of introduction orifices when the second substrate is provided on the first substrate and are formed in both ends of the concave portion, respectively.

8. The measurement device of claim 7, wherein the second substrate is formed of a transparent material.

9. The measurement device of claim 7, wherein the first substrate is formed of a semiconductor material.

10. The measurement device of claim 1, wherein a volume of the first communicating tube is smaller than one fifth of a volume of the first resistance bath, and wherein a volume of the second communicating tube is smaller than one fifth of a volume of the second resistance bath.

11. The measurement device of claim 1, wherein a surface of the flow pass has been subjected to hydrophilic processing.

12. The measurement device of claim 1, wherein each of the first and second resistors has a comb shape.

13. The measurement device of claim 3, further comprising:

   a third communicating tube connected to other part of the well than the parts of the well to which the first and second communicating tubes are connected and extending in a fourth direction different from the second and third directions;
   a third resistance bath connected to the third communicating tube; and
   a third resistor arranged in the third communicating tube or the third resistance bath.

14. The measurement device of claim 13, wherein the first and second communicating tubes are arranged so that a line extending along each said tubes extends toward said at least a microelectrode and each said tubes has an opening toward said at least a microelectrode, and
wherein the third communicating tube is arranged so that the end portion of said at least a microelectrode is located substantially on a straight line extending along the third communicating tube.

15. The measurement device of claim 1, further comprising:

   another flow pass extending in the first direction;
   another pair of introduction orifices connected to both ends of said another flow pass, respectively;
   another concave well formed in said another flow pass;
   another reference electrode formed in said another flow pass;
   at least another microelectrode formed in said another well;

another first communicating tube connected to said another well and extending in the second direction different from the first direction;

another second communicating tube connected to other part of said another well than part of said another well to which said another first communicating tube is connected;

another first resistance bath and another second resistance bath connected to said another communicating tube and said another communicating tube, respectively; and

another first resistor and another second resistor arranged in said another first communicating tube and said another second communicating tube, respectively, or in said another first resistance bath and said another second resistance bath, respectively.

16. A measurement method for measuring an electrical signal from a biological sample, the method comprising the steps of:

a) preparing a measurement device including a flow pass extending in a first direction, a pair of introduction orifices connected to both ends of the flow pass, respectively, a concave well formed in the flow pass, a reference electrode formed in the flow pass, at least a microelectrode formed in the well, a first communicating tube connected to the well and extending in a second direction different from the first direction, a second communicating tube connected to other part of the well than part of the well to which the first communicating tube is connected and extending in a third direction different from the first and second directions, first and second resistance baths connected to the first and second communicating tubes, respectively, and first and second resistors arranged in the first and second communicating tubes, respectively, or the first and second resistance baths, respectively;

b) introducing an electrolyte solution into the flow pass by reduction of a pressure in the flow pass to fill the flow pass;

c) returning the pressure in the flow pass to an atmosphere pressure;

d) arranging the biological sample in the well;

e) closing up the flow pass;

f) applying a current to the first or second resistor to bring said at least a microelectrode in contact with the biological sample; and

g) measuring change in an electrical signal from the biological sample.

17. The measurement method of claim 16, further comprising after the step f),the step h) of applying an electrical pulse to said at least a microelectrode.

18. The measurement method of claim 16 or 17, further comprising the step i) of administering a drug from one of the pair of introduction orifices.

**Patentansprüche**

1. Messvorrichtung (10) zum Messen eines elektrischen Signals von einer biologischen Probe, die umfasst:

einen Strömungsweg (9), der in einer ersten Richtung verläuft;

ein Paar Einleitöffnungen (13a, 14a), die jeweils mit beiden Enden des Strömungsweges verbunden sind;

eine konkave Vertiefung (19), die in dem Strömungsweg ausgebildet ist;

eine Bezugselektrode (2), die in dem Strömungsweg ausgebildet ist;

wenigstens eine Mikroelektrode (1), die in der Vertiefung ausgebildete ist;

eine erste Verbindungsröhre (15a), die mit der Vertiefung verbunden ist und sich in einer zweiten Richtung erstreckt, die sich von der ersten Richtung unterscheidet;

eine zweite Verbindungsröhre, die mit einem anderen Teil der Vertiefung als dem Teil der Vertiefung verbunden ist, mit dem die erste Verbindungsröhre verbunden ist, und

sich in einer dritten Richtung erstreckt, die sich von der ersten und der zweiten Richtung unterscheidet;

ein erstes und ein zweites Widerstandsbad (6a, 6b), die mit der ersten bzw. der zweiten Verbindungsröhre verbunden sind; und

einen ersten sowie einen zweiten Widerstand (3a, 3b), die in der ersten bzw. der zweiten Verbindungsröhre oder dem ersten bzw. dem zweiten Widerstandsband angeordnet sind.

2. Messvorrichtung nach Anspruch 1, wobei die erste und die zweite Verbindungsröhre so eingerichtet sind, dass sie Achsensymmetrie in Bezug auf den Strömungsweg aufweisen.

3. Messvorrichtung nach Anspruch 1, wobei die erste und die zweite Verbindungsröhre so angeordnet sind, dass eine gerade Linie, die entlang der ersten Verbindungsröhre verläuft, und eine gerade Linie, die entlang der zweiten Verbindungsröhre verläuft, einander in Richtung der wenigstens einen Mikroelektrode schneiden.

4. Messvorrichtung nach Anspruch 1, wobei die wenigstens eine Mikroelektrode einen vorstehenden Endabschnitt enthält.

**5.** Messvorrichtung nach Anspruch 1, wobei die wenigstens eine Mikroelektrode mehrfach vorhanden ist.

**6.** Messvorrichtung nach Anspruch 5, wobei eine Fläche der Bezugselektrode größer ist als eine Gesamtfläche der Vielzahl von Mikroelektroden.

**7.** Messvorrichtung nach Anspruch 1, die des Weiteren umfasst:

ein erstes Substrat; und
ein zweites Substrat, das auf dem ersten Substrat ausgebildet ist,
wobei das erste Substrat die Vertiefung, die Bezugselektrode, die Mikroelektrode, die erste und die zweite Verbindungsröhre, das erste und das zweite Widerstandsband sowie den ersten und den zweiten Widerstand enthält, und
wobei das zweite Substrat einen konkaven Abschnitt, der als der Strömungsweg dient, wenn das zweite Substrat auf dem ersten Substrat vorhanden ist, und der in der ersten Richtung verläuft, sowie Hohlräume enthält, die als die paarigen Einleitöffnungen dienen, wenn das zweite Substrat auf dem ersten Substrat vorhanden ist, und die jeweils in beiden Enden des konkaven Abschnitts ausgebildet sind.

**8.** Messvorrichtung nach Anspruch 7, wobei das zweite Substrat aus einem transparenten Material besteht.

**9.** Messvorrichtung nach Anspruch 7, wobei das erste Substrat aus einem Halbleitermaterial besteht.

**10.** Messvorrichtung nach Anspruch 1, wobei ein Volumen der ersten Verbindungsröhre kleiner ist als ein Fünftel eines Volumens des ersten Widerstandsbades, und wobei ein Volumen der zweiten Verbindungsröhre kleiner ist als ein Fünftel eines Volumens des zweiten Widerstandsbades.

**11.** Messvorrichtung nach Anspruch 1, wobei eine Oberfläche des Strömungsweges hydrophiler Behandlung unterzogen worden ist.

**12.** Messvorrichtung nach Anspruch 1, wobei der erste und der zweite Widerstand jeweils eine Kammform haben.

**13.** Messvorrichtung nach Anspruch 3, die des Weiteren umfasst:

eine dritte Verbindungsröhre, die mit dem anderen Teil der Vertiefung als denjenigen Teilen der Vertiefung verbunden ist, mit denen die erste und die zweite Verbindungsröhre verbunden sind, und die sich in einer vierten Richtung erstreckt, die sich von der zweiten und der dritten Richtung unterscheidet;
ein drittes Widerstandsbad, das mit der dritten Verbindungsröhre verbunden ist; und
einen dritten Widerstand, der in der dritten Verbindungsröhre oder dem dritten Widerstandsbad angebracht ist.

**14.** Messvorrichtung nach Anspruch 13, wobei die erste und die zweite Verbindungsröhre so angeordnet sind, dass sich eine Linie, die entlang jeder der Röhren verläuft, auf die wenigstens eine Mikroelektrode zu erstreckt, und jede der Röhren eine Öffnung zu der wenigstens einen Mikroelektrode hin hat, und wobei die dritte Verbindungsröhre so angeordnet ist, dass sich der Endabschnitt der wenigstens einen Mikroelektrode im Wesentlichen auf einer geraden Linie befindet, die entlang der dritten Verbindungsröhre verläuft.

**15.** Messvorrichtung nach Anspruch 1, die des Weiteren umfasst:

einen weiteren Strömungsweg, der in einer ersten Richtung verläuft;
ein weiteres Paar Einleitöffnungen, die jeweils mit beiden Enden des weiteren Strömungsweges verbunden sind;
eine weitere konkave Vertiefung, die in dem weiteren Strömungsweg ausgebildet ist;
eine weitere Bezugselektrode, die in dem weiteren Strömungsweg ausgebildet ist;
wenigstens eine weitere Mikroelektrode, die in der weiteren Vertiefung ausgebildet ist;
eine weitere erste Verbindungsröhre, die mit der weiteren Vertiefung verbunden ist und sich in einer zweiten Richtung erstreckt, die sich von der ersten Richtung unterscheidet;
eine weitere zweite Verbindungsröhre, die mit einem anderen Teil der weiteren Vertiefung als dem Teil der weiteren Vertiefung verbunden ist, mit dem die weitere erste Verbindungsröhre verbunden ist, und sich in einer dritten Richtung erstreckt, die sich von der ersten und der zweiten Richtung unterscheidet;
ein weiteres erstes Widerstandsbad und ein weiteres zweites Widerstandsbad, die mit der weiteren bzw. der weiteren Verbindungsröhre verbunden sind; und
einen weiteren ersten Widerstand sowie einen weiteren zweiten Widerstand, die in der weiteren ersten Verbindungsröhre bzw. der weiteren zweiten Verbindungsröhre oder dem weiteren ersten Widerstandsbad bzw. dem weiteren zweiten Widerstandsband angeordnet sind.

**16.** Messverfahren zum Messen eines elektrischen Signals von einer biologischen Probe, wobei das Verfahren die folgenden Schritte umfasst:

a) Fertigen einer Messvorrichtung, die einen Strömungsweg, der in einer ersten Richtung verläuft, ein Paar Einleitöffnungen, die jeweils mit beiden Enden des Strömungsweges verbunden sind, eine konkave Vertiefung, die in dem Strömungsweg ausgebildet ist, eine Bezugselektrode, die in dem Strömungsweg ausgebildet ist, wenigstens eine Mikroelektrode, die in der Vertiefung ausgebildet ist, eine erste Verbindungsröhre, die mit der Vertiefung verbunden ist und sich in einer zweiten Richtung erstreckt, die sich von der ersten Richtung unterscheidet. eine zweite Verbindungsröhre, die mit einem anderen Teil der Vertiefung als dem Teil der Vertiefung verbunden ist, mit dem die erste Verbindungsröhre verbunden ist, und sich in einer dritten Richtung erstreckt, die sich von der ersten und der zweiten Richtung unterscheidet, ein erstes und ein zweites Widerstandsbad, die mit der ersten bzw. der zweiten Verbindungsröhre verbunden sind, und einen ersten sowie einen zweiten Widerstand enthält, die in der ersten bzw. der zweiten Verbindungsröhre oder dem ersten bzw. dem zweiten Widerstandsband angeordnet sind;

b) Einleiten einer Elektrolytlösung in den Strömungsweg durch Verringerung eines Drucks in dem Strömungsweg, um den Strömungsweg zu füllen;

c) Zurückführen des Drucks in dem Strömungsweg auf einen atmosphärischen Druck;

d) Anordnen der biologischen Probe in der Vertiefung;

e) Schließen des Strömungsweges;

f) Anlegen eines Stroms an den ersten oder den zweiten Widerstand, um die wenigstens eine Mikroelektrode in Kontakt mit der biologischen Probe zu bringen; und

g) Messen von Änderung des elektrischen Signals von der biologischen Probe.

17. Messverfahren nach Anspruch 16, das des Weiteren nach dem Schritt f) den Schritt h) des Anlegens eines elektrischen Impulses an die wenigstens eine Mikroelektrode umfasst.

18. Messverfahren nach Anspruch 16 oder 17, das des Weiteren den Schritt i) des Verabreichens eines Medikaments über eine des Paars von Einleitöffnungen umfasst.

**Revendications**

1. Dispositif de mesure (10) permettant de mesurer un signal électrique à partir d'un échantillon biologique, comprenant :

un passage d'écoulement (9) s'étendant dans une première direction,

une paire d'orifices d'introduction (13a, 14a) reliés respectivement aux deux extrémités du passage d'écoulement,

un puits concave (19) formé dans le passage d'écoulement,

une électrode de référence (2) formée dans le passage d'écoulement,

au moins une microélectrode (1) formée dans le puits, un premier tube communicant (15a) relié au puits et s'étendant dans une deuxième direction différente de la première direction,

un deuxième tube communicant, relié à une partie du puits différente de la partie du puits à laquelle est relié le premier tube communicant, et s'étendant dans une troisième direction différente des première et deuxième directions,

des premier et deuxième bains résistifs (6a, 6b) reliés respectivement aux premier et deuxième tubes communicants, et

des première et deuxième résistances (3a, 3b) disposées respectivement dans les premier et deuxième tubes communicants, ou bien respectivement dans les premier et deuxième bains résistifs.

2. Dispositif de mesure selon la revendication 1, dans lequel les premier et deuxième tubes communicants sont disposés de façon à présenter une symétrie linéaire par rapport au passage d'écoulement.

3. Dispositif de mesure selon la revendication 1, dans lequel les premier et deuxième tubes communicants sont disposés de telle sorte qu'une ligne droite s'étendant le long du premier tube communicant et une ligne droite s'étendant le long du deuxième tube communicant se coupent l'une avec l'autre vers ladite ou lesdites microélectrodes.

4. Dispositif de mesure selon la revendication 1, dans lequel ladite ou lesdites microélectrodes incluent une partie finale saillante.

5. Dispositif de mesure selon la revendication 1, dans lequel il est prévu que ladite ou lesdites microélectrodes soient multiples.

6. Dispositif de mesure selon la revendication 5, dans lequel la surface de l'électrode de référence est plus grande que la surface totale de la pluralité de microélectrodes.

7. Dispositif de mesure selon la revendication 1, comprenant en outre :

un premier substrat, et
un second substrat formé sur le premier substrat,

dans lequel le premier substrat inclut le puits, l'électrode de référence, la microélectrode, les premier et deuxième tubes communicants, les premier et deuxième bains résistifs et les première et deuxième résistances, et

dans lequel le second substrat inclut une partie concave qui sert de passage d'écoulement lorsque le second substrat est ménagé sur le premier substrat et s'étend dans la première direction, ainsi que des cavités qui servent de paire d'orifices d'introduction lorsque le second substrat est ménagé sur le premier substrat et qui sont respectivement formées aux deux extrémités de la partie concave.

**8.** Dispositif de mesure selon la revendication 7, dans lequel le second substrat est formé d'un matériau transparent.

**9.** Dispositif de mesure selon la revendication 7, dans lequel le premier substrat est formé d'un matériau semiconducteur.

**10.** Dispositif de mesure selon la revendication 1, dans lequel le volume du premier tube communicant est inférieur à un cinquième du volume du premier bain résistif, et

dans lequel le volume du deuxième tube communicant est inférieur à un cinquième du volume du deuxième bain résistif.

**11.** Dispositif de mesure selon la revendication 1, dans lequel la surface du passage d'écoulement a été soumise à un traitement hydrophile.

**12.** Dispositif de mesure selon la revendication 1, dans lequel chacune des première et deuxième résistances présente une forme de peigne.

**13.** Dispositif de mesure selon la revendication 3, comprenant en outre :

un troisième tube communicant, relié à une partie du puits différente des parties du puits auxquelles sont reliés les premier et deuxième tubes communicants, et

s'étendant dans une quatrième direction différente des deuxième et troisième directions,

un troisième bain résistif relié au troisième tube communicant, et

une troisième résistance disposée dans le troisième tube communicant ou dans le troisième bain résistif.

**14.** Dispositif de mesure selon la revendication 13, dans lequel les premier et deuxième tubes communicants sont disposés de telle sorte qu'une ligne s'étendant le long de chaque dit tube s'étende vers ladite ou lesdites microélectrodes, et chaque dit tube possède une ouverture vers ladite ou lesdites microélectrodes, et

dans lequel le troisième tube communicant est disposé de telle sorte que la partie finale de ladite ou desdites microélectrodes soit sensiblement située sur une ligne droite s'étendant le long du troisième tube communicant.

**15.** Dispositif de mesure selon la revendication 1, comprenant en outre :

un autre passage d'écoulement s'étendant dans la première direction,

une autre paire d'orifices d'introduction reliés respectivement aux deux extrémités dudit autre passage d'écoulement,

un autre puits concave formé dans ledit autre passage d'écoulement,

une autre électrode de référence formée dans ledit autre passage d'écoulement,

au moins une autre microélectrode formée dans ledit autre puits,

un autre premier tube communicant relié au dit autre puits et s'étendant dans la deuxième direction différente de la première direction,

un autre deuxième tube communicant relié à une autre partie dudit autre puits, différente de la partie dudit autre puits à laquelle est relié ledit autre premier tube communicant,

un autre premier bain résistif et un autre seconde bain résistif reliés respectivement au dit autre premier tube communicant et au dit autre deuxième tube communicant, et

une autre première résistance et une autre deuxième résistance disposées respectivement dans ledit autre premier tube communicant et dans ledit autre deuxième tube communicant, ou bien respectivement dans ledit autre premier bain résistif et dans ledit autre deuxième bain résistif.

**16.** Procédé de mesure permettant de mesurer un signal électrique à partir d'un échantillon biologique, le procédé comprenant les étapes consistant à :

a) préparer un dispositif de mesure incluant un passage d'écoulement s'étendant dans une première direction, une paire d'orifices d'introduction reliés respectivement aux deux extrémités du passage d'écoulement, un puits concave formé dans le passage d'écoulement, une électrode de référence formée dans le passage d'écoulement, au moins une microélectrode formée dans le puits, un premier tube communicant relié au puits et s'étendant dans une deuxième direction différente de la première direction, un deuxième tube communicant relié à une partie du puits différente de la partie du puits à laquelle est relié le premier tube communicant et s'étendant dans une troisième direction différente des première et deuxième directions, des premier et deuxième bains résistifs reliés respectivement aux premier et deuxième tubes communicants et des première et deuxième résistances disposées respectivement dans les premier et deuxième tubes communicants, ou bien respectivement dans les premier et deuxième bains résistifs,

b) introduire une solution d'électrolyte dans le passage d'écoulement par réduction de pression dans le passage d'écoulement afin de remplir le passage d'écoulement,

c) faire revenir la pression dans le passage d'écoulement à la pression atmosphérique,

d) disposer l'échantillon biologique dans le puits,

e) obturer le passage d'écoulement,

f) appliquer un courant à la première ou à la seconde résistance pour amener ladite ou lesdites microélectrodes en contact avec l'échantillon biologique, et

g) mesurer toute modification dans un signal électrique issu de l'échantillon biologique.

17. Procédé de mesure selon la revendication 16, comprenant en outre, après l'étape f), l'étape h) consistant à appliquer une impulsion électrique à ladite ou aux dites microélectrodes.

18. Procédé de mesure selon la revendication 16 ou 17, comprenant en outre l'étape i) consistant à administrer un médicament à partir de l'un de la paire d'orifices d'introduction.

# FIG. 1

# FIG. 2

FIG. 3

EP 1 460 415 B1

FIG. 4A

6a

7        12                    15a        9        8    9

                                                         1

6b              15b

FIG. 4B

6a

                              15a    12          8    9
7

                                                      1

6b              15b

FIG. 4C

6a

                          15a  12        8    9
7

                                              1

6b              15b

FIG. 5

EP 1 460 415 B1

FIG. 6A

FIG. 6B

# FIG. 7

FIG. 8

# FIG. 9

EP 1 460 415 B1

## FIG. 10

St1 — Measuring intracellular potential

St2 — Injecting stimulation solution into flow pass

St3 — Measuring intracellular potential

St4 — Cleaning inside of well

FIG. 11

FIG. 12

# FIG. 13

**EP 1 460 415 B1**

**REFERENCES CITED IN THE DESCRIPTION**

**Patent documents cited in the description**

- JP 9289886 A

- JP 10337177 A